# EUROPEAN PATENT APPLICATION

(11) **EP 4 806 403 A1**
(43) Date of publication of application: **16.09.2026**
(21) Application number: 24888441.3
(22) Date of filing: 07.10.2024
(51) Int. Cl.: A61F 7/03

(54) **HEATING ELEMENT**

(30) Priority: 08.11.2023 JP 2023190970
(71) Applicant: Kobayashi Pharmaceutical Co., Ltd., Chuo-ku Osaka-shi, Osaka 541-0045 (JP)
(72) Inventor: SONODA, Goro, Ibaraki-shi, Osaka 567-0057 (JP); ISHIDA, Yuri, Ibaraki-shi, Osaka 567-0057 (JP)
(74) Representative: Vossius & Partner Patentanwälte Rechtsanwälte mbB
(86) International application number: PCT/JP2024/035726
(87) International publication number: WO 2025/100142

(57) **Abstract**

Provided is a heat generator capable of reducing peeling even when attached at or near a movable part of a human body. A heat generator 10 comprises a body part 1 having a storage part 18 thereon, the storage part 18 being formed by joining outer peripheries of a front sheet part 11 and a back sheet part 12; a pair of adhesive parts 2 each having an adhesive layer 3 on one surface thereof, the adhesive parts 2 each extending from a corresponding end edge of the body part 1 on both sides in a horizontal, i.e., left-right, direction; and a heat-generating material 4 housed in the storage part 18. In the corresponding end edge of the body part 1 on both sides in the horizontal direction, a region on one side with respect to a center line C1 that passes through the center of the body part 1 in a vertical direction and that is parallel to the horizontal direction includes a connection region at which the body part 1 is connected to a corresponding one of the adhesive parts 2, while a region on the other side with respect to the center line C1 is entirely a non-connection region at which the body part 1 is not connected to a corresponding one of the adhesive parts 2.

## Description

### Technical Field

The present invention relates to a heat generator.

### Background Art

PTL 1 discloses a conventional heat generator (referred to as a "heating tool" in PTL 1). The heating tool disclosed in PTL 1 is attached to, for example, the back of the neck (nape). The heating tool includes an inner bag formed in a bag shape by joining a first sheet and a second sheet, a heating layer positioned in the inner bag, and an adhesive layer provided on one surface of the inner bag. The adhesive layer is formed entirely over the one surface of the inner bag and is formed in a rectangular shape in plan view. The adhesive layer allows the heating tool to adhere to the user's skin.

### Citation List

### Patent Literature

PTL 1: JP2019-37775A

### Summary of Invention

### Technical Problem

As described above, in the heating tool of PTL 1, the adhesive layer is provided over the entire surface of the inner bag. Therefore, if a user with the heating tool of PTL 1 attached to a movable part, such as the back of the neck, moves their head by performing movements, such as looking upward, the head applies a force to the heating tool from the upper side of the heating tool and from the human body side, which leads to a problem in that the heating tool is easily peeled off.

The present invention has been made in view of the above circumstances. An object of the present invention is to provide a heat generator capable of reducing peeling even when attached at or near a movable part of a human body.

### Solution to Problem

A heat generator according to one aspect of the present invention comprises a body part having a storage part thereon, the storage part being formed by joining outer peripheries of a front sheet part and a back sheet part; a pair of adhesive parts each having an adhesive layer on one surface thereof, the adhesive parts each extending from a corresponding end edge of the body part on both sides in a horizontal, i.e., left-right, direction; and a heat-generating material housed in the storage part. A region on one side with respect to a center line that passes through the center of the body part in a vertical direction and that is parallel to the horizontal direction includes a connection region at which the body part is connected to a corresponding one of the adhesive parts, while a region on the other side with respect to the center line is entirely a non-connection region at which the body part is not connected to a corresponding one of the adhesive parts.

### Advantageous Effects of Invention

The heat generator according to the above aspect of the present invention has an advantage of being able to reduce peeling even when it is attached at or near a movable part of a human body.

### Brief Description of Drawings

Fig. 1 is a schematic perspective view showing a state of use of the heating tool according to the present embodiment.
Fig. 2 is a schematic rear view of the heating tool according to the present embodiment, as viewed from the back surface.
Fig. 3 is a cross-sectional view taken along line A-A in Fig. 2.
Fig. 4(A) is a schematic front view of a heating tool according to a modification example. Fig. 4(B) is a schematic front view of a heating tool according to a further modification example. Fig. 4(C) is a schematic front view of a heating tool according to a further modification example.
Fig. 5 is a schematic front view of a heating tool according to a further modification example.

### Description of Embodiments

### Embodiments

The heat generator 10 according to the present embodiment is described in detail below with reference to the drawings.

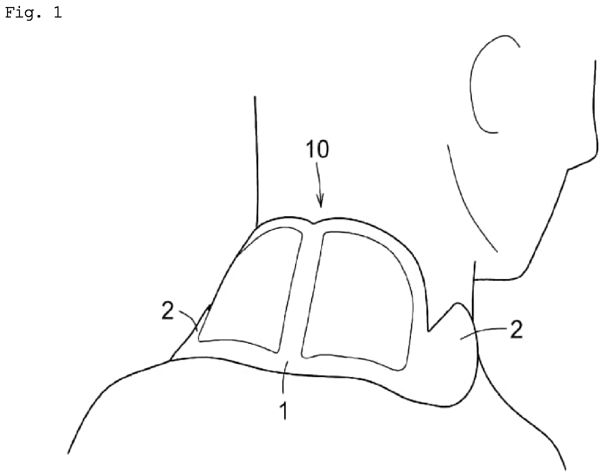

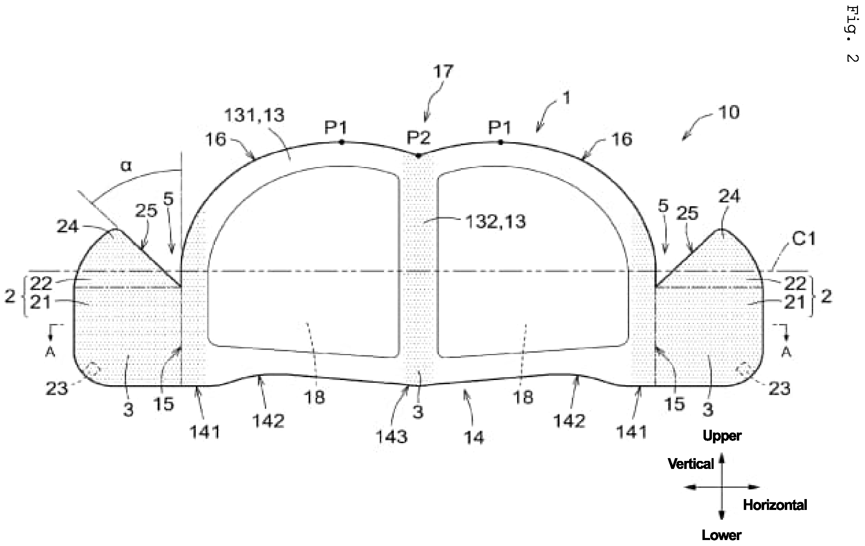

As shown in Fig. 1, the heat generator 10 is a heat generator 10 to be attached to a movable part of a user, such as the nape of the neck (posterior cervical region). The heat generator 10 according to the present embodiment comprises a body part 1 having heat-generating regions, and a pair of adhesive parts 2 each extending from a corresponding end edge of the body part 1 on both sides in a horizontal direction. As shown in Fig. 2, in the corresponding end edge of the body part 1 on both sides in the horizontal direction, at least a region on one side with respect to a center line C1 that passes through the center of the body part 1 in a vertical direction and that is parallel to the horizontal direction includes a connection region at which the body part 1 is connected to a corresponding one of the adhesive parts 2, while a region on the other side with respect to the center line C1 is entirely a non-connection region at which the body part 1 is not connected to a corresponding one of the adhesive parts 2.

Therefore, for example, when the heat generator 10 according to the present embodiment is attached to the nape of the neck such that one side of the body part 1 is positioned on the lower side and the other side is positioned on the upper side, as shown in Fig. 1, the body part 1 is unlikely to peel off even if the head pushes the body part 1 outward while the user moves their head. Specifically, when an external force is applied to the body part 1 from the upper side and from the human body side, portions on the upper side with respect to the center line C1 of the body part 1 deflect in a direction away from the human body; however, since the adhesive parts 2 are each connected to the body part 1 only on the lower side with respect to the center line C1, the movement of the body part 1 is unlikely to be transmitted to the adhesive parts 2, and the adhesive parts 2 are thus unlikely to peel off. The heat generator 10 according to the present embodiment is thus capable of reducing peeling even when it is attached at or near a movable part.

For the heat generator 10 according to the present embodiment, the movable part of a user that serves as a target site for attachment may be, for example, the knees, back of the knees, elbows, shoulders, and shoulder blades in addition to the nape of the neck. In the present embodiment, the nape of the neck of a user is used as an example of the movable part for attachment.

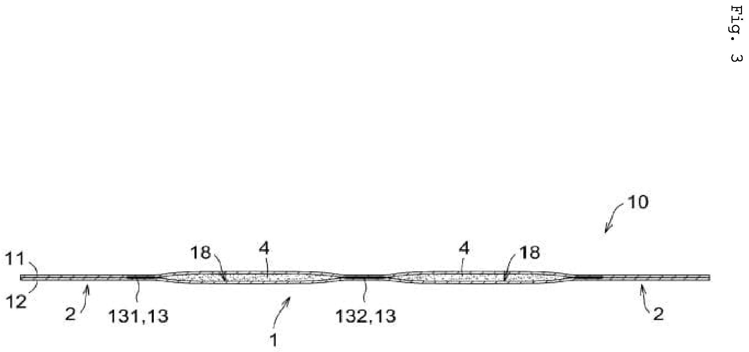

As shown in Fig. 2, the heat generator 10 according to the present embodiment comprises the body part 1 having storage parts 18, the pair of adhesive parts 2 each having an adhesive layer 3, and heat-generating materials 4 each housed in the storage parts 18 (Fig. 3). The heat generator 10 has flexibility. The heat generator 10 according to the present embodiment is a disposable body warmer and may otherwise be a reusable body warmer.

In the following description, for convenience of explanation, the longitudinal direction of the body part 1 is defined as a "horizontal direction," and the transverse direction of the body part 1 is defined as a "vertical direction," as shown in Fig. 2. The horizontal direction and the vertical direction are orthogonal to each other. In the vertical direction, one side is defined as the "lower side," and the other side is defined as the "upper side." A direction orthogonal to the vertical direction and the horizontal direction is defined as the "thickness direction." Additionally, in the heat generator 10, a surface facing the user's skin is referred to as a "back surface," and a surface on the opposite side is referred to as a "front surface." However, these definitions of directions are merely for the purpose of explanation and are not intended to specify the mode of use of the heat generator 10.

In the present specification, "parallel" includes not only cases in which two straight lines, planes, etc. (referred to below as "straight lines etc.") do not intersect even if extended, but also cases in which the angle formed by the intersection of two straight lines etc. is within a range of 10° or less. The term "orthogonal" means a case in which two straight lines etc. intersect at an angle within a range of 90° ± 10°. However, "orthogonal" includes cases in which two straight lines etc. would intersect if extended even when they do not directly intersect each other.

The phrase "the center of the body part in the vertical direction" as used in the present specification means a single central point on a line segment of the minimum length extending from an imaginary straight line that passes through the point furthest to one side (lower side) of the body part in the vertical direction and that is parallel to the horizontal direction, to an imaginary straight line that passes through the point furthest to the other side (upper side) in the vertical direction and that is parallel to the horizontal direction. Similarly, "the center of the body part in the horizontal direction" means a single central point on a line segment of the minimum length extending from an imaginary straight line that passes through the extreme end point on a first side (e.g., the left side) of the body part in the horizontal direction and that is parallel to the vertical direction, to an imaginary straight line that passes through the extreme end point on a second side (e.g., the right side) in the horizontal direction and that is parallel to the vertical direction. The "first side" of the body part and the "second side" of the body part as used herein refer to opposite sides to each other in the horizontal direction.

### Body Part 1

In the heat generator 10, the body part 1 is a portion in which heat-generating regions are surrounded by a joint part 13. The body part 1 is formed by overlapping a front sheet part 11 and a back sheet part 12, and joining their outer peripheries with the joint part 13. Portions surrounded by the joint part 13 and between the front sheet part 11 and the back sheet part 12 include bag-shaped storage parts 18, in which heat-generating materials 4 are each housed.

The outer peripheral edge of the body part 1 is composed of a lower edge portion 14 extending along the horizontal direction, a pair of side edge portions 15, and a pair of arc portions 16.

The lower edge portion 14 includes a pair of linear portions 141 parallel to the horizontal direction and separated from each other, a pair of recessed portions 142 each connected to the inner sides of the linear portions 141 and recessed upward, and a substantially V-shaped protruding portion 143 formed between the pair of recessed portions 142. The recessed portions 142 are formed in a curved shape and are smoothly connected to the linear portions 141 and the protruding portion 143. The tip portion of the protruding portion 143 is positioned at the center of the body part 1 in the horizontal direction and protrudes downward relative to the recessed portions 142.

The side edge portions 15 each extend in the vertical direction. To at least a portion of the side edge portion 15, a corresponding one of the adhesive parts 2 is connected. In the present embodiment, a portion of the side edge portion 15 is formed integrally with the adhesive part 2, and the connecting portion connected to the adhesive part 2 serves as an imaginary boundary line.

The arc portions 16 each connect one of the side edge portions 15 and the upper end of the body part 1 at the center in the horizontal direction. The arc portion 16 is a curved line having an arc in at least a portion thereof. The curved line of the arc portion 16 does not necessarily have the same curvature from one end to the other end. The uppermost point of the arc portion 16 may be referred to as "vertex P1." The portion between a pair of vertices P1 of the pair of arc portions 16 is constricted and is formed in a substantially V-shape. The portion constricted in the V-shape may be referred to as a "constricted portion 17." The constricted portion 17 according to the present embodiment is positioned at the center part of the body part 1 in the horizontal direction. The tip of the constricted portion 17 may be referred to as a "valley point P2".

The end edges of the body part 1 in the horizontal direction as used herein mean edges having a component parallel to the vertical direction along the end edges. In the present embodiment, in the outer peripheral edge, the end edges of the body part 1 in the horizontal direction are each composed of the side edge portion 15 and a portion of the arc portion 16 in the range from the end that is connected to the side edge portion 15 to the vertex P1.

The joint part 13 is a part in which the front sheet part 11 and the back sheet part 12 are joined. In the present embodiment, the joint part 13 is formed by welding (heat sealing). However, the joint part 13 is not limited to being formed by welding and may be formed by, for example, bonding, sewing, compression bonding, or the like.

The joint part 13 includes a peripheral joint part 131 and an intermediate joint part 132. The peripheral joint part 131 is formed along the outer peripheral edge of the front sheet part 11 and the outer peripheral edge of the back sheet part 12. The peripheral joint part 131 is formed with substantially the same width over its entire length. The intermediate joint part 132 is provided inside the peripheral joint part 131. In this manner, the intermediate joint part 132 divides the region inside the peripheral joint part 131 into a plurality of regions. In the present embodiment, the intermediate joint part 132 extends along the vertical direction of the body part 1 at the center in the horizontal direction. However, a plurality of intermediate joint parts 132 may be provided, for example, at regular intervals in the horizontal direction.

The storage parts 18 according to the present embodiment are surrounded by the peripheral joint part 131 and the intermediate joint part 132. The body part 1 has a plurality of storage parts 18; however, the number of the storage parts 18 may be one. That is, the intermediate joint part 132 may be omitted.

### Adhesive Part 2

In the heat generator 10, adhesive parts 2 are portions that are to be adhered to a user. An adhesive layer 3 is provided on one surface (back surface) of each of the adhesive parts 2 (in Fig. 2, the adhesive layer 3 is indicated with dots). The adhesive parts 2 each extend outwardly in the horizontal direction from a corresponding one of the side edge portions 15 of the body part 1. The adhesive parts 2 each include an adhesive body part 21 protruding in the horizontal direction from the corresponding one of the side edge portions 15, and an extension part 22 protruding from the adhesive body part 21. The extension part 22 is not connected to the body part 1. In the present specification, being not connected may be referred to as "non-connection."

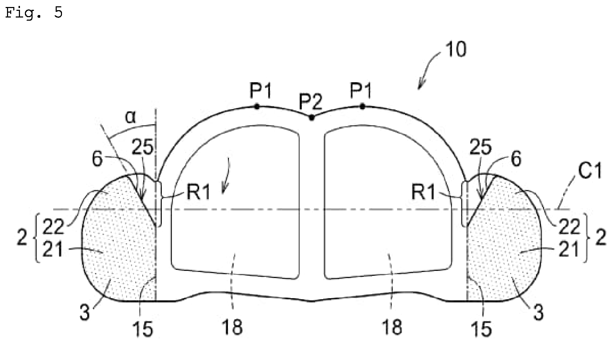

The adhesive body parts 21 are each connected to the corresponding end edge of the body part 1 in the horizontal direction only within a region on the lower side with respect to the center line C1 (one side in the vertical direction). In the present embodiment, the adhesive body parts 21 are each connected to the corresponding side edge portion 15 within a specific range including the lower end thereof. That is, in the corresponding end edge of the body part 1 in the horizontal direction, at least a region on the upper side with respect to the center line C1 (the other side in the vertical direction) is entirely a non-connection region. The "non-connection region" refers to a region at which the adhesive part 2 is not connected to the end edge of the body part 1 in the horizontal direction. The region is separated from the extension part 22 by, for example, a slit (see, for example, Fig. 5), a notch, or the like. In the present embodiment, the non-connection region is separated from the adhesive part 2 by a triangular notch 5. The non-connection region may also be included in a region on the lower side with respect to the center line C1.

Due to the presence of the notch 5, the adhesive part 2 has an edge 25 facing the non-connection region. The angle α of the edge 25 of the adhesive part 2 facing the non-connection region with respect to the vertical direction is preferably 0° or more and 50° or less, and more preferably 15° or more and 45° or less. This configuration reduces deflection or the like caused by a force from the movable part, and the adhesive parts 2 can be made more resistant to peeling.

The term "center line C1" as used in the present specification means an imaginary straight line that passes through the center of the body part 1 in the vertical direction and that is parallel to the horizontal direction. The center line C1 in the present embodiment is a straight line that passes through the center point between the uppermost point (vertex P1) and the lowermost point (a point on the lower edge portion 14 or the tip of the protruding portion 143) of the body part 1 in the vertical direction.

In the present embodiment, in each of the side edge portions 15 of the body part 1, a portion on the upper side is the non-connection region while the other portion is a connection region to which the adhesive body part 21 is connected. The distance from the lower edge portion 14 of the body part 1 to the upper end of the connection region is preferably 50% or less, more preferably 45% or less, and even more preferably 40% or less, relative to the maximum distance of the body part 1 in the vertical direction. On the other hand, the lower limit of the distance from the lower edge portion 14 of the body part 1 to the upper end of the connection region is preferably 25% or more, more preferably 30% or more, and even more preferably 35% or more, relative to the maximum distance of the body part 1 in the vertical direction. By setting the distance from the lower edge portion 14 of the body part 1 to the upper end of the connection region to be 25% or more and 50% or less relative to the maximum distance of the body part 1 in the vertical direction, the movement of the body part 1 is less likely to be transmitted to the adhesive parts 2, and the adhesive parts 2 and the body part 1 are thus not easily separated.

The "maximum distance of the body part 1 in the vertical direction" as used herein means the minimum distance from an imaginary straight line that passes through the point furthest to one side (lower side) of the body part in the vertical direction and that is parallel to the horizontal direction, to an imaginary straight line that passes through the point furthest to the other side (upper side) in the vertical direction and that is parallel to the horizontal direction.

The horizontal length of each of the adhesive body parts 21 is preferably 0.8 times or more, more preferably 1.0 times or more, the vertical length of the connection region. On the other hand, the horizontal length of each of the adhesive body parts 21 is preferably 2 times or less, more preferably 1.8 times or less, and even more preferably 1.5 times or less, the vertical length of the connection region.

The extension part 22 protrudes from each of the adhesive body parts 21 and expands the adhesive area of each of the adhesive body parts 21. In the present embodiment, the extension part 22 protrudes upward from each of the adhesive body parts 21. The area of the extension part 22 may be smaller or larger than that of each of the adhesive body parts 21.

The upper end of each of the extension parts 22 (the upper end 24 of each of the adhesive parts) is preferably positioned on the upper side with respect to the center line C1. This allows the adhesive area to be expanded without expanding the adhesive parts 2 in the horizontal direction. However, it is preferable that the upper end of each of the extension parts 22 (the upper end 24 of each of the adhesive parts) is positioned below the uppermost point of the body part 1 (vertex P1). This configuration prevents the adhesive parts 2 from adhering to the user's scalp hair while expanding the adhesive area. In the present embodiment, in the vertical direction, the upper end of each of the extension parts 22 (the upper end 24 of each of the adhesive parts) is positioned between the vertex P1 of the body part 1 and the center line C1.

The adhesive parts 2 are each preferably configured by overlapping the front sheet part 11 and the back sheet part 12. It is preferable that the front sheet part 11 and the back sheet part 12 constituting each of the adhesive parts 2 are partially joined to each other. That is, in each of the adhesive parts 2, the front sheet part 11 and the back sheet part 12 are joined at one point of a dot-like joint part 23 at a rounded corner portion formed by a lower edge and a side edge. Compared to an adhesive part 2 in which the front sheet part 11 and the back sheet part 12 are joined over the entire surface, the above configuration imparts higher flexibility and enables easy deformation; therefore, the user is less likely to feel pain when moving their head with the heat generator being attached, and furthermore, adhesiveness to the skin can also be improved.

The area of the adhesive layers 3 in the adhesive parts 2 is preferably 15% or more, more preferably 20% or more, and even more preferably 25% or more, of the area of the body part 1. On the other hand, the area of the adhesive layers 3 in the adhesive parts 2 is preferably 65% or less, more preferably 60% or less, and even more preferably 55% or less, of the area of the body part 1. When the area of the adhesive layers 3 in the adhesive parts 2 is 25% or more of the area of the body part 1, high adhesive strength to the user can be maintained. When the area of the adhesive layers 3 in the adhesive parts 2 is 30% or less of the area of the body part 1, adhesion to scalp hair can be prevented and the burden on the skin can be alleviated. The "area of the adhesive layers 3 in the adhesive parts 2" as used herein means the total area of the adhesive layers 3 provided on a plurality of the adhesive parts 2.

The adhesive layer 3 is provided at least on the adhesive parts 2. The adhesive layer 3 may also be provided on the body part 1 in addition to the adhesive parts 2. In the present embodiment, the adhesive layer 3 is also provided on portions of the intermediate joint part 132 and the peripheral joint part 131 in addition to the adhesive parts 2. However, the adhesive layer 3 is preferably provided outside the sites corresponding to the storage parts 18. This configuration can prevent the adhesive strength from decreasing between the adhesive layer 3 and the skin even when the temperature increases at the sites corresponding to the storage parts 18 and the user perspires at these sites. The area of the adhesive layer 3 provided on the body part 1 is preferably 20% or less, and more preferably 15% or less, relative to the area of the body part 1. This configuration can reduce the sense of discomfort when the heat generator 10 is attached at or near a movable part. If the area of the adhesive layer 3 provided on the body part 1 is too large, the adhesiveness of the heat generator 10 to the user becomes too high, which increases the sense of discomfort when attached to a movable part.

In the present embodiment, the adhesive layer 3 is present continuously over the entire surface of each of the adhesive parts 2 on one side; however, for example, the adhesive layer 3 may be present in a striped or dotted (polka-dot) pattern at regular intervals.

The adhesive layer 3 is not particularly limited. Examples include adhesives, such as acrylic adhesives, vinyl adhesives, vinyl acetate adhesives, polyvinyl acetal adhesives, vinyl chloride adhesives, cellulose adhesives, chloroprene adhesives, nitrile rubber adhesives, styrene rubber adhesives, silicone rubber adhesives, polysulfide adhesives, terpene resins, and water-soluble rosin. From the viewpoint of ensuring an appropriate level of adhesive strength that is neither too strong nor too weak, a styrene rubber adhesive is preferably used.

The front sheet part 11 and the back sheet part 12 constituting the body part 1 and the adhesive parts 2 are preferably resin films. The resin used for the resin films is preferably a thermoplastic resin. Examples of the thermoplastic resin include polyethylene, polypropylene, polyester, polyamide, polyurethane, polystyrene, polyvinyl alcohol, polyvinyl chloride, polyvinylidene chloride, polycarbonate, and ethylene-vinyl acetate copolymer. These resins may be used alone or in a combination of two or more.

From the viewpoint of, for example, improving skin feel, sweat absorption, and enhancing the anchoring properties of the glue, it is preferable that the back sheet part 12 be provided with a woven fabric or non-woven fabric on the outside of the resin film. Examples of the fiber material for the woven fabric or non-woven fabric include natural fibers, such as cotton, hemp, silk, and paper; semi-synthetic fibers, such as rayon and acetate; synthetic fibers, such as nylon, vinylon, polyester, acrylic, polyethylene, polypropylene, polyvinyl chloride, and polybutylene terephthalate; and mixed fibers of these fibers. Among these, the fiber material is preferably nylon, polyester, polypropylene, or the like from the viewpoint of improving skin feel. These fiber materials may be used alone or in a combination of two or more. The basis weight of the woven fabric or non-woven fabric is not particularly limited as long as it can prevent the heat-generating material 4 from leaking out of the body part 1, and may be, for example, 20 g/m² or more and 70 g/m² or less.

However, the body part 1 and the adhesive parts 2 do not have to be a laminate of a resin film and a woven fabric or non-woven fabric, and may be composed of a single resin film, or a single woven fabric or non-woven fabric.

The body part 1 has breathability. Since the resin film is a porous film or a resin film in which a plurality of vent holes are formed, the body part 1 has a plurality of vent holes and is thus capable of ventilation through the plurality of vent holes.

### Heat-generating Material 4

The heat-generating material 4 is a material that is housed in the storage part 18, as shown in Fig. 3, and that is capable of generating heat. The heat-generating material 4 according to the present embodiment comes into contact with air and undergoes oxidation, thus generating heat. The heat-generating material 4 is a powdery material.

Examples of the heat-generating material 4 include oxidizable metals, activated carbon, carbon black, water-retaining agents, metal salts (e.g., sodium chloride), and water. Oxidizable metals radiate heat of oxidation reaction and include, for example, one, two, or more powders or fibers selected from the group consisting of iron, aluminum, zinc, manganese, magnesium, and calcium. Of these, iron powder is preferably used from the viewpoint of handling, safety, production costs, storage, and stability. Iron powder is, for example, one, two, or more members selected from the group consisting of reduced iron powder and atomized iron powder. Examples of water-retaining agents include wood flour, vermiculite, diatomaceous earth, perlite, silica gel, alumina, and water-absorbing resin. The heat-generating material 4 for use may be a powdery composition used in conventional disposable body warmers as is, and is not particularly limited in the present invention.

The heat-generating material 4 for use may be a material other than those that generate heat by coming into contact with air. For example, the heat-generating material 4 for use may be a material that generates heat by being irradiated with microwaves in a microwave oven or the like (e.g., ceramic powder, such as ferrite and red beans) or a chemical liquid that generates heat using heat of coagulation.

### Usage Example

As shown in Fig. 1, the heat generator 10 configured as described above is attached such that the adhesive parts 2 adhere to the user's skin, with the movable part (head) of the user positioned on the upper side of the body part 1. In this state, when the user moves their head, an external force is applied to the body part 1 from the upper side and from the human body side according to the user's movement. This causes the portion of the body part 1 on the upper side with respect to the center line C1 to deflect in a direction away from the human body; however, since the adhesive parts 2 are connected only to the lower side with respect to the center line C1, the movement of the body part 1 is unlikely to be transmitted to the adhesive parts 2, and the adhesive parts 2 are thus unlikely to peel off. The heat generator 10 according to the present embodiment can thus reduce peeling even when it is attached at or near a movable part of a user.

When the heat generator 10, which is less likely to peel off, is attached to the nape of the neck, the nape of the neck can be effectively warmed. Thereby, stiffness in the shoulders and neck can be relieved, and any pain can be alleviated.

### Modification Examples

The embodiment above is merely one of various embodiments of the present invention. The embodiment can be modified in various ways according to the design or the like as long as the object of the present invention is achieved. Modification examples of the embodiment are listed below. The modification examples described below can be suitably combined for use.

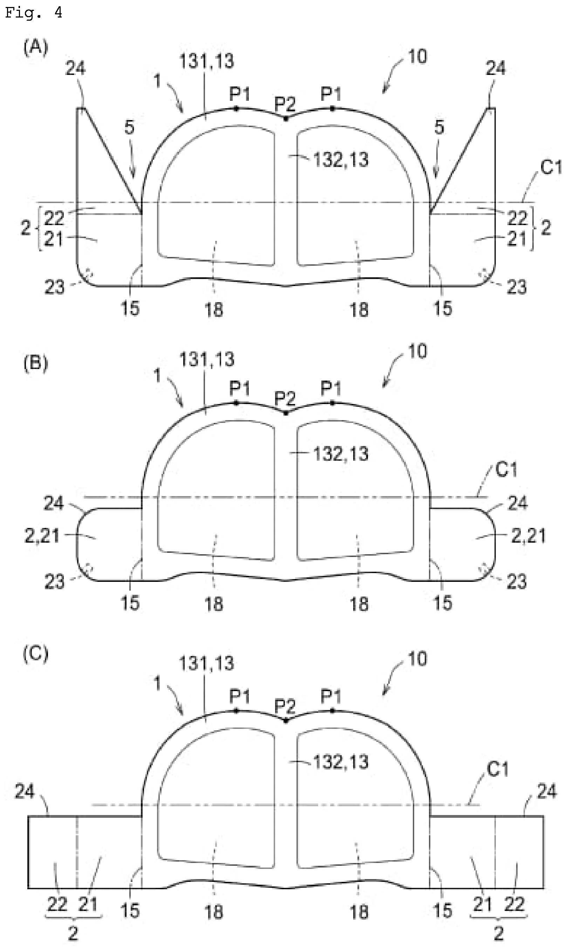

In the above embodiment, the upper end 24 of each of the adhesive parts 2 is positioned below the vertices P1 of the body part 1 on the upper side; however, as shown in Fig. 4(A), the upper end 24 of each of the adhesive parts 2 may be positioned above the vertices P1 of the body part 1 on the upper side. In this case, for example, when the heat generator 10 is attached at or near a movable part without scalp hair or the like, the problem of scalp hair or the like adhering to the adhesive parts 2 does not occur, and the adhesive area can be expanded while making the length in the horizontal direction as short as possible.

In the above embodiment, the adhesive parts 2 each have the extension part 22; however, as shown in Fig. 4(B), the extension part 22 may be omitted. When the extension part 22 is not provided, the adhesive area is reduced, and the adhesive parts 2 are relatively easily peeled off, compared to the above embodiment. However, the heat generator 10 of this modification example can be suitably attached to a site at which the movement of a movable part is small. The heat generator 10 of this modification example has a resistance to peeling while reducing the burden on the skin. In this modification example, the upper end 24 of each of the adhesive parts 2 is positioned below the vertices P1 of the body part 1 on the upper side.

In the above embodiment, the extension part 22 protrudes upward from each of the adhesive body parts 21; however, as shown in Fig. 4(C), the extension parts 22 may protrude in the horizontal direction. In this modification example, the upper end 24 of each of the adhesive parts 2 is positioned below the vertices P1 of the body part 1 on the upper side. In this case, a large adhesive area can be ensured, and the adhesive parts 2 can be placed as far away from the scalp hair as possible when the heat generator is attached at or near the nape of the neck as the movable part.

In the above embodiment, the non-connection region is separated from the adhesive part 2 by the notch 5; however, as shown in Fig. 5, the non-connection region may be separated from the adhesive part 2 by a slit 6. In the modification example shown in Fig. 5, a substantially triangular piece surrounded by the slit 6 and the side edge portion 15 is connected to a region R1 of the side edge portion 15 on the upper side; however, since the region R1 is separated from the adhesive part 2 by the slit 6, the region R1 here is referred to as a "non-connection region." Note that the adhesive layer 3 is not provided on the triangular piece.

In each of the adhesive parts 2, an edge 25 facing the non-connection region is formed by the slit 6. The angle of the slit 6 with respect to the vertical direction (that is, the angle α of the edge 25 of the adhesive part 2 facing the non-connection region with respect to the vertical direction) is preferably 0° or more and 50° or less, and more preferably 15° or more and 45° or less. This configuration enables the adhesive parts 2 to be more resistant to peeling.

In the above embodiment or modification examples, the non-connection region is separated from the adhesive part 2 by the slit 6 or the notch 5; however, the non-connection region may be separated from the adhesive part 2 by a combination of the notch 5 and the slit 6.

In the above embodiment, the adhesive part 2 has a configuration in which the front sheet part 11 and the back sheet part 12 are overlapped; however, the adhesive part 2 does not have to be, for example, configured by using two layers and may be configured by using only the front sheet part 11 or the back sheet part 12, not both.

In the above embodiment, the longitudinal direction of the body part 1 is defined as a horizontal direction and the transverse direction is defined as a vertical direction; however, the vertical direction and the horizontal direction of the body part 1 may be of the same length. The body part 1 may have a shape such as a rectangular shape, a square shape, an elliptical shape, or a circular shape.

In the above embodiments, in the vertical direction, one side is referred to as the "lower side" and the other side is referred to as the "upper side"; however, one side may be referred to as a "first side" and the other side may be referred to as a "second side."

In the above embodiments, the adhesive parts 2 are described as an example configured to directly adhere to the user's skin; however, the adhesive parts 2 may adhere to user's clothing.

### Effects

As described above, the heat generator 10 according to a first aspect comprises a body part 1 having a storage part 18 thereon, the storage part 18 being formed by joining outer peripheries of a front sheet part 11 and a back sheet part 12; a pair of adhesive parts 2 each having an adhesive layer 3 on one surface thereof, the adhesive parts 2 each extending from a corresponding end edge of the body part 1 on both sides in the horizontal direction; and a heat-generating material 4 housed in the storage part 18. In the corresponding end edge of the body part 1 on both sides in the horizontal direction, a region on one side with respect to a center line C1 that passes through the center of the body part 1 in the vertical direction and that is parallel to the horizontal direction includes a connection region at which the body part 1 is connected to a corresponding one of the adhesive parts 2, while a region on the other side with respect to the center line C1 is entirely a non-connection region at which the body part 1 is not connected to a corresponding one of the adhesive parts 2.

According to this aspect, even when the heat generator 10 is attached such that the adhesive parts 2 adhere to a user with a movable part positioned on the other side of the body part 1, and a force is applied from the movable part to the body part 1, the movement of the body part 1 is unlikely to be transmitted to the adhesive parts 2 because the adhesive parts 2 are connected only to one side with respect to the center line C1, making the adhesive parts 2 resistant to peeling. The heat generator 10 can thus reduce peeling even when it is attached at or near a movable part.

In a second aspect of the heat generator 10 according to the first aspect, the non-connection region is separated from the adhesive part 2 by a slit 6. According to this aspect, the adhesive parts 2 can be made more resistant to peeling with a simple configuration.

In a third aspect of the heat generator 10 according to the first or second aspect, the non-connection region is separated from the adhesive part 2 by a triangular notch 5. According to this aspect, portions other than the adhesive parts 2 can be cut off, which can reduce cases in which portions other than the adhesive parts 2 come into contact with the user's skin and cause injury to the skin.

In a fourth aspect of the heat generator 10 according to the second or third aspect, an angle α of an edge 25 of the adhesive part 2 facing the non-connection region with respect to the vertical direction is 15° or more and 45° or less. According to this aspect, the adhesive parts 2 can be made more resistant to peeling.

In a fifth aspect of the heat generator 10 according to any one of the first to fourth aspects, an end of the adhesive part 2 on the other side in the vertical direction is positioned closer to the one side than an end of the body part 1 on the other side in the vertical direction. According to this aspect, for example, when the heat generator 10 is attached at or near the head with scalp hair as the movable part of a user, the adhesive parts 2 are prevented from adhering to the user's scalp hair.

In a sixth aspect of the heat generator 10 according to any one of the first to fifth aspects, in the vertical direction, the end of the adhesive part 2 on the other side in the vertical direction is positioned between the end of the body part 1 on the other side in the vertical direction and the center line C1. According to this aspect, for example, when the heat generator 10 is attached at or near the head with scalp hair as the movable part of a user, the adhesive parts 2 are prevented from adhering to the user's scalp hair while ensuring a large adhesive area to the user.

In a seventh aspect of the heat generator 10 according to any one of the first to sixth aspects, the area (total area) of the adhesive layers 3 in the pair of adhesive parts 2 is 15% or more of the area of the body part 1. According to this aspect, an appropriate adhesive strength to the user can be ensured.

In eighth aspect of the heat generator 10 according to any one of the first to seventh aspects, the adhesive part 2 includes a portion of the front sheet part 11 and a portion of the back sheet part 12, and the portion of the front sheet part 11 and the portion of the back sheet part 12 are partially joined. According to this aspect, the adhesive parts 2 are more easily deformed compared with an embodiment of the adhesive part 2 in which the front sheet part 11 and the back sheet part 12 are joined over the entire surface, and when, for example, the heat generator is caused to adhere to the user's skin, the adhesiveness to the user's skin can be improved.

### Description of Reference Numerals

10: Heat generator
1: Body part
11: Front sheet part
12: Back sheet part
13: Joint part
15: Side edge portion
18: Storage part
2: Adhesive part
24: Upper end of the adhesive part (end of the adhesive part on the other side in the vertical direction)
25: Edge facing the non-connection region
3: Adhesive layer
4: Heat-generating material
5: Notch
6: Slit
C1: Center line
α: Angle of the edge facing the non-connection region with respect to the vertical direction

## Claims

1. A heat generator comprising:
a body part having a storage part thereon, the storage part being formed by joining outer peripheries of a front sheet part and a back sheet part;
a pair of adhesive parts each having an adhesive layer on one surface thereof, the adhesive parts each extending from a corresponding end edge of the body part on both sides in a horizontal direction; and
a heat-generating material housed in the storage part,
wherein in the corresponding end edge of the body part on both sides in the horizontal direction, a region on one side with respect to a center line that passes through the center of the body part in a vertical direction and that is parallel to the horizontal direction includes a connection region at which the body part is connected to a corresponding one of the adhesive parts, while a region on the other side with respect to the center line is entirely a non-connection region at which the body part is not connected to a corresponding one of the adhesive parts.

2. The heat generator according to claim 1, wherein the non-connection region is separated from the adhesive part by a slit.

3. The heat generator according to claim 1, wherein the non-connection region is separated from the adhesive part by a triangular notch.

4. The heat generator according to claim 2 or 3, wherein an angle of an edge of the adhesive part facing the non-connection region with respect to the vertical direction is 15° or more and 45° or less.

5. The heat generator according to any one of claims 1 to 3, wherein an end of the adhesive part on the other side in the vertical direction is positioned closer to the one side than an end of the body part on the other side in the vertical direction.

6. The heat generator according to claim 5, wherein the end of the adhesive part on the other side in the vertical direction is positioned between the end of the body part on the other side in the vertical direction and the center line.

7. The heat generator according to any one of claims 1 to 3, wherein an area of the adhesive layers in the pair of adhesive parts is 150 or more of an area of the body part.

8. The heat generator according to any one of claims 1 to 3, wherein the adhesive part includes a portion of the front sheet part and a portion of the back sheet part, and the portion of the front sheet part and the portion of the back sheet part are partially joined.
